# EUROPÄISCHE PATENTSCHRIFT

(11) **EP 1 567 210 B1**
(45) Veröffentlichungstag und Bekanntmachung des Hinweises auf die Patenterteilung: **28.05.2008**
(21) Anmeldenummer: 03785738.0
(22) Anmeldetag: 04.12.2003
(51) Int. Cl.: A61M 1/30

(54) **Verfahren und Vorrichtung zum Separieren von Vollblut unter Schwerkraft in ein Erythrozytenkonzentrat und zellfreies oder thrombozytenhältiges Plasma**
Method and device for separating whole blood into an erythrocyte concentrate and cell-free or thrombocyte-containing plasma under gravitational force
Procédé et dispositif pour séparer du sang entier par gravité pour obtenir un concentré d'érythrocytes et du plasma dépourvu de cellules ou contenant des thrombocytes

(30) Priorität: 04.12.2002 DE 10256848
(43) Veröffentlichungstag der Anmeldung: 31.08.2005
(73) Patentinhaber: Latza, Sibylle, 66386 St. Ingbert (DE)
(72) Erfinder: Latza, Sibylle, 66386 St. Ingbert (DE)
(74) Vertreter: Luderschmidt, Schüler & Partner
(86) Internationale Anmeldenummer: PCT/EP2003/013705
(87) Internationale Veröffentlichungsnummer: WO 2004/050145

(56) Entgegenhaltungen:
- EP-A- 0 266 683
- WO-A-02/13888
- DE-U- 29 516 471
- US-A- 4 964 847

## Beschreibung

Die Erfindung betrifft ein Verfahren zum Separieren von Vollblut in Leukozyten depletiertes Erythrozytenkonzentrat und Plasma sowie eine Vorrichtung zur Durchführung dieses Verfahrens.

Bei der Vollblutseparation wird von Blutspendern abgenommenes Vollblut in die einzelnen Blutkomponenten separiert. Hierbei handelt es sich überwiegend um Erythrozytenkonzentrate oder Plasmafraktionen, die zellfrei oder thrombozytenhaltig sein können.

Zur Auftrennung von Vollblut benötigt man heute entweder speziell eingerichtete Herstellungsräume, Hochleistungszentrifugen, Bluttrenngeräte, wie Plasmafilter, sowie speziell geschultes Personal oder aber man setzt Plasmaseparatoren zur direkten Gewinnung einzelner oder kombinierter Blutkomponenten ein.

In Folge der gestiegenen Qualitätsanforderungen bzw. gesetzlicher Regelungen und der inzwischen entwickelten Hochleistungsblutseparatoren ist es einem Transfusionsmediziner oder kleineren Kliniken praktisch unmöglich geworden, auf diese Weise Blutprodukte herzustellen und derart produzierte Blutprodukte im Markt anzubieten. Sinnvoll wäre es deshalb, eine einfache Trennung von Blutkomponenten auch in diesem Bereich zu ermöglichen, ohne den hohen Qualitätsstandard zu verlassen. Des Weiteren wäre es sinnvoll, solche Blutprodukte auch ohne hohen technischen und somit teuren Aufwand auf dieser Basis herzustellen.

Aus der DE 33 02 383 A1 ist ein Verfahren und eine Vorrichtung zur Gewinnung von Blutplasma bekannt, wobei in vivo Vollblut einem Patienten entnommen wird, das im Anschluß in einem Plasmafilter in ein Erythrozyten-Konzentrat und eine Plasmafraktion aufgeteilt wird.

In einer weiteren Ausgestaltung wird das Erythrozyten-Konzentrat durch das Plasmafilter unter erneuter Abtrennung einer weiteren Plasmafraktion zum Patienten zurückgeführt.

Es wird zwar eine Doppelfraktionierung von Plasma durchgeführt, das Erythrozyten-Konzentrat wird jedoch dem Blutspender unmittelbar ohne Zwischenlagerung wieder zurückgegeben.

Die EP 349 188 beschreibt ein Verfahren zum Separieren von Blut in Blutkomponenten, sowie eine Separatoreinheit zur Gewinnung dieser Blutkomponenten. Nach diesem Verfahren wird Blut aus einem Vollblutbehälter zunächst durch ein Filter geleitet, das sowohl Leukozyten als auch Blutplättchen entfernt. Im Anschluss daran wird das filtrierte Blut in einem Primärbeutel gesammelt, der dann einer Zentrifugation zur Auftrennung des Bluts in eine Plasmafraktion und einem Erythrozyten-Konzentrat unterzogen wird. Das Plasma wird hierbei über eine Plasmaleitung in einen weiteren Plasmabeutel geleitet.

Wie bereits vorstehend erläutert, ist dieses Verfahren aufgrund des Einsatzes einer Zentrifuge technisch aufwendig. Außerdem kann das Plasma nicht vollständig getrennt werden, da ansonsten die Gefahr der Vermischung mit Erythrozyten stattfinden kann.

Die US 5,527,472 beschreibt ebenfalls ein geschlossenes System zur Trennung von Vollblutbestandteilen mittels Zentrifugation. Bei diesem Verfahren findet zunächst in einem ersten Beutel die Zentrifugation unter Auftrennung in Plasma und ein Erythrozyten-Konzentrat statt, das dann mit einer Substitutionslösung vermischt wird. Dieses Gemisch wird im Anschluss daran in einem Leukozyten entfernenden Filter von Leukozyten entfernt, so dass ein Erythrozyten-Konzentrat mit Substituatlösung erhalten wird, das jedoch noch einen hohen Anteil an Plasma enthält.

Auch dieses Verfahren ist technisch aufwendig, wobei im Erythrozyten-Konzentrat ein hoher Anteil von Blutplasma zurückbleibt.

WO 02/13888 offenbart eine Filteranordnung zur Auftrennung von Blut in Plasma und zelluläre Bestandteile unter Verwendung des Filters des Typs Mikro PSE-TF 10 der Akzo Faser AG. Die Anordnung ist luft- keimdicht abgeschlossen. Vor der Auftrennung des Vollblutswerden gegebenenfalls Leukozyten und Thrombozyten abgetrennt. Durch Umkehr der Flussrichtung durch die Filteranordnung nach dem erstem Durchgang kann in der Erythrozytenfraktion ein Hämatokritwert von mindestens 80 erhalten werden.

WO 02/13888 offenbart nicht die gleichzeitige Auftrennung von Vollblut in ein Erythrozytenkonzentrat und ein thrombozytenhaltiges Plasma.

Infolgedessen liegt der Erfindung die Aufgabe zugrunde, ein technisch einfaches Verfahren zum Separieren von Vollblut in ein Erythrozytenkonzentrat und ein thrombozytenhaltiges Plasma vorzusehen, bei dem möglichst wenig Plasma in Erythrozyten-Konzentrat verbleibt und das unmittelbar am Spenderort, insbesondere ohne zeitliche Verzögerung nach der Entnahme, durchgeführt werden kann.

Die Lösung der Aufgabe erfolgt durch die Merkmale des Anspruchs 1.

Weiterhin wird diese Aufgabe unter Einsatz der Vorrichtung gemäß Anspruch 7 gelöst.

Weitere Vorteile der Erfindung ergeben sich aus der nachfolgenden Beschreibung.

Das erfindungsgemäße Verfahren setzt ein bei einer Vollblutfraktion, die entweder unmittelbar nach Spende oder auch nach mehrstündiger Zwischenlagerung behandelt werden soll. Gemäß der gelagerten Ausführungsform lässt sich einem Spender entnommenes Blut von Leukozyten in einem Leukozytenfilter besser behandeln, da nach einer Lagerung von etwa 30 Minuten oder mehr der Leukozytenabtrenngrad erheblich besser und somit zufriedenstellend ist.

Andererseits kann die Abtrennung der Leukozyten und anderer Zellen unmittelbar nach Entnahme durchgeführt werden.

Nachstehend wird derartiges Vollblut als "gelagert" oder "nicht gelagert" bezeichnet.

Gemäß einer ersten Ausführungsform erfolgt die Leukozytenabtrennung unmittelbar hinter dem vollblutenthaltenden Beutel, somit stromauf des Plasmafilters.

Andererseits ist der Ort dieser Leukozytenabtrennung nicht kritisch und kann auch stromab des Filters, demzufolge unmittelbar vor dem Beutel liegen, der das endgültige Erythrozyten-Konzentrat aufnimmt (2. Ausführungsform). Schließlich kann auch die Leukozytenabtrennung stromauf des Beutels, also in dessen Zulauf- oder Überführungsleitung vorgenommen werden (3. Ausführungsform).

Die Vollblutfraktion wird also durch ein Filter geleitet, mit dem sowohl Mikroaggregate als auch Leukozyten entfernt werden. Solche Filter sind dafür bekannt, dass sie 99,9% und mehr Leukozyten aus Vollblut entfernen, so dass das Vollblut praktisch von Leukozyten depletiert ist.

In einem weiteren Schritt wird ein derart von Leukozyten und depletiertes Vollblut mittels eines Plasmafilters in ein erstes Erythrozytenkonzentrat und eine erste thrombozytenhaltige Plasmafraktion mittels einer mikroporösen Membran aufgetrennt, die die Erythrozyten zurückhält, jedoch sämtliche flüssige Bestandteile und Thrombozyten durchlässt. Zuvor ist das Plasmafilter mit einer Kochsalzlösung gefüllt worden, die als Vorlauf der Blutbehandlung abgetrennt wird.

Bei diesem Separationsschritt wird der Hämatokrit, also der Volumenanteil der Erythrozyten am Vollblut auf wenigstens 45-55, vorzugsweise wenigstens 50% angehoben. Infolgedessen werden je nach Provenienz üblicherweise ca. 10 bis 20 Vol.-% des Vollbluts als thrombozytenhaltige Plasmafraktion am Plasmaausgang des Plasmafilters entnommen.

Die Plasmafiltration in dieser Stufe erfolgt mittels Schwerkraft, wobei der hydrostatische Druck max. 1,5 - 2,5 m Ws (0,15-0,25 bar) zwischen dem Ausgang des Vollblutbeutels und dem Eingang des Plasmafilters beträgt. Vorzugsweise beträgt der Eingangsdruck zwischen Blutbeutel und Plasmafilter mindestens 0,7, vorzugsweise etwa 1,5 m Ws (Wassersäule) (entsprechend 0,07 - 0,15 bar).

Diese relativ mäßige Druckdifferenz gewährleistet, dass keine Hämolyse der Erythrozyten an der Membran stattfindet.

Die zum Einsatz kommenden Plasmafilter haben üblicherweise kapillare Membranen, also Hohlfasermembranen, und weisen eine Membranoberfläche von 0,1 - 0,5 m² auf.

Übliche Membranmaterialien sind Polymere vom EVA, PVA-Typ, Zellulosederivate, Polyolefine (Polypropylen), PAN, PA, Polyester, Polysulfone und dergleichen. Bevorzugt sind Polysulfone oder Polypropylen.

Eine mittlere Porengröße von kleiner als 2 µm, vorzugsweise zwischen 1 und 1,5 µm wird gewählt, um Thrombozyten (mittlerer Durchmesser etwa 0,5 - 1 µm) von den restlichen korpuskulären Bestandteilen zusammen mit Plasma abzutrennen.

Zur Abtrennung aller zellulären Bestandteile von der Plasmafraktion liegen die üblichen Porengrößen zwischen 0,03 und 0,4 µm, je nach Abhängigkeit der eingesetzten Membran. Wesentlich an der Porengröße ist lediglich, dass die zellulären Bestandteile des Bluts von der Membran bei der Plasmafiltration zurückgehalten werden. Einsetzbare Filter sind beispielsweise Plasmafilter der Firma Dideco der Bezeichnung "HEMAPLEX".

Vorteilhafterweise werden solche Plasmafilter bereits mit einer sterilen, pyrogenfreien Kochsalzlösung gefüllt eingesetzt und sind somit mit Vollblut benetzbar, insbesondere wenn sie aus einem hydrophoben Material bestehen.

Derartige Filter weisen ein Gehäuse auf, das durch die semipermeable Membran in zwei Kammern geteilt ist, nämlich eine Erythrozyten und andere Zellen (Leucozyten) führende Kammer mit einem ersten Anschluss und einem zweiten Anschluss sowie eine Plasma Thrombozyten und Plasmaproteine führende Kammer mit einem Plasmaauslass.

Am zweiten Anschluss der Erythrozytenkammer (Auslass gemäß der ersten Erythrozyten-Anreicherung) wird das auf mindestens 45-55, vorzugsweise 50% Hämatokrit angereicherte Erythrozyten-Konzentrat in einem ersten Erythrozyten-Konzentratbeutel aufgefangen.

Der Beutel zum Auffangen des ersten Erythrozyten-Konzentrats ist dabei üblicherweise etwas unterhalb des Plasmafilters angeordnet, üblicherweise ca. 0,1 - 0,3, vorzugsweise bis 0,2 m Ws.

Insgesamt beträgt die Druckdifferenz zwischen dem Ausgang der VollblutKonserve und dem Eingang des Beutels für das erste Erythrozytenkonzentrat vorzugsweise zwischen 1 und 1,2 m Ws.

Der Beutel zum Auffangen des zellfreien Plasmas ist ebenfalls nach dem Gesetz der Schwerkraft unterhalb des Filters angeordnet, üblicherweise bis zu 1 m Ws, vorzugsweise zwischen 0,75 - 0,9 m Ws.

Dieser gesamte hydrostatische Druck ca. 1,5-2,5 m, vorzugsweise 1,8 - 2 m Ws. reicht zu einer wirksamen Separation von Plasma aus, ohne dass eine Hämolyse-Gefahr bestehen würde.

Um möglichst wenig Plasma im endgültigen Erythrozytenkonzentrat zu erhalten, wird dieses erneut einer Plasmafiltration mit dem gleichen Abtrennsystem unterzogen, d.h. es wird der gleiche Plasmafilter jedoch mit umgekehrter Flussrichtung durchflossen, wobei die gleichen Anschlussleitungen mit dem gleichen Blutbeutel eingesetzt werden. Infolgedessen strömt das erste Erythrozytenkonzentrat aus dem nunmehr oberhalb (gravimetrisch gesehen) des Plasmafilters angeordneten Beutel, wobei die Aufhängehöhe etwa derjenigen der Vollblutkonserve entspricht, d.h. der hydrostatische Druck auf den Plasmafilter entspricht demjenigen der ersten Auftrennung von Vollblut. Infolgedessen wird am ersten Anschluss (Einlass in der ersten Stufe) ein zweites Erythrozytenkonzentrat aufgefangen, dessen Hämatokritwert mindestens 60 %, vorzugsweise 70% beträgt. Durch diesen zweiten Separationsvorgang wird also ein Erythrozyten-Konzentrat erhalten, bei dem etwa 2/3, vorzugsweise 3/4 des ursprünglich vorhandenen Plasmas und mehr aus dem Vollblut separiert worden ist.

Auch dieser zweite Beutel wird ähnlich wie der erste Beutel gravimetrisch unterhalb des Plasmafilters bei dieser zweiten Konzentrierung angeordnet, wobei die Höhenverhältnisse derjenigen der ersten Konzentrierung der Erythrozyten bzw. der Separation des Plasmas entsprechen.

Diesen Vorgang kann man nun noch einmal wiederholen, bis sich ein Hämatokrit von 80-90 % ergibt und damit einerseits die Fließfähigkeit des Blutes sehr deutlich reduziert wird und andererseits ca. 15 % des ursprünglichen Plasma im Erythrozytenkonzentrat zurückbleiben. Da die Restmenge an Plasma für einen Teil der Nebenwirkungen der Transfusion von Erythrozytenkonzentraten verantwortlich ist, ergibt sich hier ein Vorteil, der in diesem Umfang durch Zentrifugation nicht zu erreichen ist.

Zur Aufbewahrung eines derart gewonnenen Erythrozytenkonzentrats ist vorteilhaft in dem zweiten Konzentratbeutel eine Substituatlösung (Additivlösung) vorgesehen, wie sie üblicherweise Erythrozytenkonzentraten zugesetzt wird. Es handelt sich dabei um wässrige Lösungen, die Natriumchlorid, Adenin, Glukose (SAG-Lösung) enthalten, die gegebenenfalls mit Mannit (SAG-M) vermischt sind. Derartige Lösungen sind - wie festgestellt - als Additivsysteme für die Speicherung von Erythrozytenkonzentraten bekannt.

Erfindungsgemäß kann die gemäß dem zweiten Konzentrierungsschritt gewonnene Lösung auf einen Hämatokrit eingestellt werden, der gleich oder größer 70% ist.

Die Verbindung mit Additivlösung führt dann beispielsweise zu einem Blutsystem mit einem üblichen Hämatokrit (40 % und mehr).

Nach erfolgter Trennung wird der Plasmabeutel nach einer weiteren Ausgestaltung abgeklemmt und steht dann als zellfreies Plasma zum Einfrieren und zur Lagerung zur Verfügung. Andererseits kann auch die Plasmakammer des Plasmafilters unter sterilen Bedingungen belüftet werden, was zur Entleerung führt, so dass eine Mehrausbeute von etwa 50 ml hochwertigem Plasma zur Verfügung steht. Diese Ausbeute ist um etwa 20 % höher als bei der üblichen Zentrifugationsmethode.

Der Erythrozytenbeutel enthält nach dem Abklemmen Restplasma von etwa 15 %. Dieses Erythrozytenkonzentrat wird mit einer additiven Lösung - wie vorstehend erläutert - versetzt und anschließend gut durchgemischt. So kann man beispielsweise ein fließfähiges Produkt bei einem Hämatokrit von etwa 60 % erhalten, das als leuko-/thrombo-/plasma-depletiertes Erythrozytenkonzentrat zur Verfügung steht.

In einer weiteren Ausgestaltung des erfindungsgemäßen Verfahrens kann Erythrozytenkonzentrat, das noch Restplasma enthält, mit einer Kochsalzlösung gewaschen werden, wobei zunächst sterile Kochsalzlösung dem Erythrozytenkonzentrat zugesetzt wird, das anschließend erneut durch das Plasmafilter filtriert wird. So kann man beispielsweise 500 - 1000 ml 0,9 %-ige NaCl-Lösung nach sterilem Konnektieren dem Erythozytenkonzentrat zugeben, woraufhin eine gründliche Durchmischung erfolgt. Im Anschluss daran wird die erhaltene Mischung so lange durch das Plasmafilter geschickt, bis erneut ein Hämatokrit von etwa 85 % erreicht ist. Die Kochsalzlösung bzw. ein diese Lösung erhaltenes Gemisch wird in einem Leerbeutel aufgefangen. Es wird erneut Additivlösung zugesetzt, wonach das Produkt als leuco-/thrombo-/plasma-depletiertes Erythrozytenkonzentrat zur Verfügung steht, d.h. das Konzentrat wird nach dieser Verfahrensstufe im wesentlichen von Plasma freigewaschen.

Das erfindungsgemäße Verfahren kann auch zur Herstellung von Thrombozytenkonzentraten unter gleichzeitiger Gewinnung von Erythrozytenkonzentraten eingesetzt werden. Hierzu wird ein Plasmafilter mit einer Membran eingesetzt, deren Porengröße (etwa 2 µm oder kleiner) so gewählt ist, dass sie zwar Thrombozyten durchlässt, Erythrozyten und Leukozyten jedoch zurückhält. Man kann nach drei Filtrationspassagen wiederum ein Erythrozytenkonzentrat mit einem Hämatokrit von etwa 85 % und eine thrombozytenreiche Plasmafraktion erhalten.

Das thrombozytenreiche Plasma kann in einer weiteren Ausführungsform weiter konzentriert werden, wobei dieses Plasma durch ein übliches Plasmafilter (Porengröße max. 0,3 µm) gefiltert wird, das nun die Thrombozyten zurückhält. Durch wiederholte Trennung von beispielsweise 300 ml Ausgangsplasma lassen sich dann 250 ml zellfreies Plasma und etwa 15 ml thrombozytenreiches Plasma erzeugen.

Im übrigen entfernt man aus dem erhaltenen Erythrozytenkonzentrat mit einem üblichen Leukodepletionsfilter die Leukozyten, wobei vorteilhafterweise zur Fließbarmachung Additivlösung dem Konzentrat hinzugefügt worden ist.

Das gesamte Abtrennsystem ist in sich steril abgeschlossen, so dass ein zellfreies Plasmaprodukt und ein Erythrozytenkonzentrat hergestellt werden, die den Qualitätsvorgaben des Paul Ehrlich-Institutes in Deutschland für solche Produkte genügen.

Das erfindungsgemäße Verfahren wird folgendermaßen durchgeführt:

Ca. ein halber Liter Spenderblut wird entweder mindestens eine halbe Stunde in einem Blutbeutel gelagert oder direkt nach der Spende durch ein Filter geschickt, der Mikroaggregate sowie mindestens 99 % der Leukozyten entfernt. Im Anschluss daran unterzieht man dieses Spenderblut als Vollblut der erfindungsgemäßen Plasmaseparation unter Herstellung eines Erythrozytenkonzentrats, das allenfalls ¼ des ursprünglich vorliegenden Plasma enthält.

Gemäss einer weiteren Ausführungsform (Waschen des Konzentrats) kann der Plasmaanteil im Erythrozytenkonzentrat durch Waschen mit einer Waschlösung, vorzugsweise einer Kochsalzlösung, auf etwa 0,5 % oder weniger gesenkt werden.

Um die Blutkonserve möglichst ohne Nebenwirkungsgefahr (Schüttelfrost, Fieber oder Schock) einem Patienten verabreichen zu können, wird das erhaltene Erythrozytenkonzentrat zuvor von Leukozyten sowie Mikroaggregaten filtriert. Dies geschieht - wie vorstehend erläutert - entweder unmittelbar stromab oder stromauf der Vollblutkonserve oder aber vor dem Einlass des endgültigen Erythrozytenkonzentrat-Beutels.

Die Filtration des Erythrozytenkonzentrats von Thrombozyten kann vor dem Einlass des endgültigen Erythrozytenkonzentrat-Beutels erfolgen.

Die Behandlungstemperatur entspricht in etwa der Raumtemperatur. Üblicherweise wird Vollblut mit der Temperatur eingesetzt, die sich unmittelbar nach der Zwischenlagerungszeit ergibt. Diese kann auch zwischen 23 und 35°C liegen.

Der Hämatokrit des Vollbluts schwankt in Abhängigkeit von seiner Herkunft (Mann / Frau) und liegt üblicherweise zwischen 36 und 45 %.

Die Laufzeit, innerhalb der die erste Plasmafiltration stattfindet, hängt ab vom Filtrationsdruck bzw. der Sogwirkung und liegt üblicherweise zwischen 15 Minuten und 45 Minuten, vorteilhafterweise etwa bei einer halben Stunde.

Es stellen sich nach der ersten Filtration Hämatrokritwerte oberhalb 50 %, vorteilhafterweise oberhalb 52 - 57 % ein.

Nach der ersten Plasmaseparation wird der das erste Erythrozytenkonzentrat enthaltene Beutel, der unterhalb des Plasmafilters hängt, in einer Position aufgehängt, die oberhalb des Plasmafilters ist, so dass unter Drehung des Beutels das erste Erythrozytenkonzentrat in umgekehrter Richtung durch den Plasmafilter zurückfließt.

Die zweite Separationszeit ist gewöhnlich kürzer als die erste und beträgt etwa 15 - 40 Minuten, üblicherweise um 15-20 Minuten.

Ausgangs des Plasmafilters stellen sich in der zweiten Stufe Hämatokritwerte zwischen 65 und 75 %, vorteilhaft oberhalb 70 %, teilweise oberhalb 73 % ein.

Weiterhin kann auch eine dritte Filtration durchgeführt werden, die zu einer Anhebung des Hämatokrit auf ca. 80 % und mehr führt.

Dieses zweite/dritte Erythrozytenkonzentrat wird zur Konservierung und auch zur Einstellung für einen Patienten auf einen Hämatokrit mit Hilfe einer Additivlösung eingestellt (SAG oder SAG-M), wobei der eingestellte Hämatokrit etwa 40 - 45 % beträgt.

Als Material für Beutel / Leitungen kommen die üblichen in der Medizintechnik eingesetzten Polymeren (PE, PP etc.) in Frage, die einerseits flexibel und verschiebbar, andererseits gut zu sterilisieren und optisch klar sind.

Die Beispiele erläutern die Erfindung. Es zeigen
- Figur 1: einen ersten schematischen Aufbau der Plasmaseparationsanordnung zur Herstellung des ersten Erythrozytenkonzentrats gemäss einer ersten Ausführungsform,
- Figur 2: einen weiteren schematischen Aufbau der Anordnung gemäß Figur 1 zur Herstellung des zweiten Erythrozytenkonzentrats,
- Figur 3: eine Plasmaseparationsanordnung ähnlich derjenigen von Figur 1 oder 2, jedoch ohne Leukozytenfilter im Zulauf gemäß einer zweiten Ausführungsform und
- Figur 4: ein Schema einer 3-stufigen Plasmafiltration mit der Anordnung gemäß Figur 3.

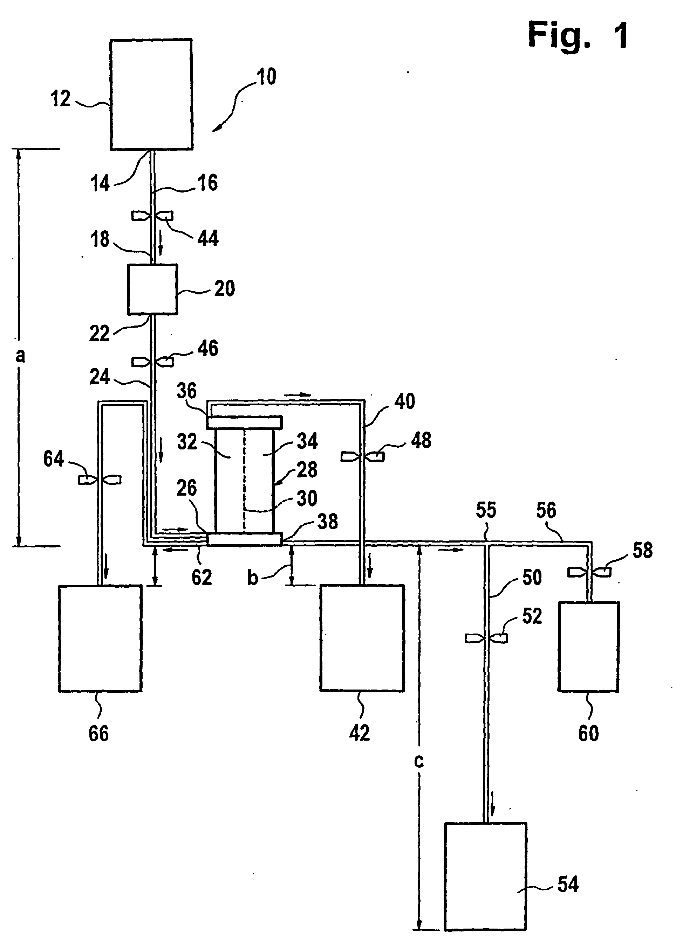

In Figur 1 ist mit 10 eine Anordnung zur Trennung von Vollblut in Plasma und Erythrozyten-Konzentrat als erste Ausführungsform gezeigt. Sie weist einen Vollblutbeutel 12 auf, der üblicherweise einem halben Liter Vollblut aufnimmt. Dieses Vollblut ist mit einer Antikoagulanz-Lösung versetzt, beispielsweise einer ACD oder CBD Lösung. Es handelt sich hier um übliche Lösungen auf der Basis von Glucose, Trinatriumzitrat und Zitronensäure, die in einer solchen Menge vorgelegt werden, dass eine Koagulation von Blut innerhalb des Trennsystems unterbleibt.

An den Vollblutbeutel 12 ist an dessen Auslass 14 eine erste flexible Leitung 16 angeschlossen, deren anderes Ende 18 mit einem Leukozyten und Mikroaggregate entfernenden Filter 20 verbunden ist. Vom Ausgang 22 des Filters 20 geht eine zweite Leitung 24ab, die mit dem ersten Anschluss 26 eines Plasmafilters 28 verbunden ist. Dieser Plasmafilter 28 ist durch eine Membran 30 in eine Blutkammer 32 und eine Plasmakammer 34 geteilt. Gegenüber dem ersten Anschluss 26, der in die Blutkammer 32 mündet, befindet sich ein zweiter Anschluss 36 an der Blutkammer, während von der Plasmakammer 34 ein Plasmaanschluss 38 abgeht. Vom zweiten Anschluss 36 geht eine dritte Leitung 40 ab, die mit einem zweiten Blutbeutel 42 zur Aufnahme eines ersten Erythrozyten-Konzentrats verbunden ist.

Die jeweiligen Leitungen 16, 24 und 40 verfügen über Klemmen 44, 46 und 48, mit denen die flexiblen Leitungen geöffnet bzw. geschlossen werden können, üblicherweise Rollklemmen.

Vom Plasmaanschluss 38 geht eine vierte Leitung 50 als Plasmaleitung ab, die ebenfalls mit einer Klemme 52 abgeklemmt werden kann. Das Ende der vierten Leitung 50 mündet in einen Plasmabeutel 54.

Stromauf der Klemme 52 verzweigt sich die vierte Leitung 50 an einem Verzweigungspunkt 55 in eine Abzweigleitung 56, in die ebenfalls eine Klemme 58 zum Abklemmen der Abzweigleitung eingeschaltet ist. Die Abzweigleitung 56 selbst ist an ihrem Ende mit einem Auffangbeutel 60 verbunden, der - wie nachstehend erläutert - eine Fülllösung für das Plasmafilter 28 aufnehmen soll.

Diese Abzweigleitung 56 muss nicht notwendigerweise von der Leitung 50 abzweigen. Sie kann vielmehr auch direkt mit dem Plasmaanschluss 38 verbunden sein.

Schließlich geht vom ersten Anschluss des Plasmafilters 26 eine fünfte Leitung 62 ab, in die ebenfalls eine Klemme 64 eingeschaltet ist. Das andere Ende der fünften Leitung 62 ist dabei mit einem dritten Blutbeutel 66 verbunden, der das zweite, endgültige Erythrozyten-Konzentrat aufnimmt.

Dieser dritte Beutel 66 weist bereits eine fertige sterile Blutverdünnungs-Lösung als Additiv-Lösung, beispielsweise eine SAG-Lösung auf.

In Figur 1 ist der Plasmafilter 28 hochkant angeordnet, d. h. der erste Anschluss 26 liegt unterhalb des zweiten Anschlusses 36, d. h. es erfolgt eine Strömung gegen die Schwerkraft, wenn Blut vom Anschluss 26 durch die Blutkammer 32 zum zweiten Anschluss 36 geführt wird, andererseits in Richtung der Schwerkraft, wenn die Strömungsrichtung sich umkehrt, also von Anschluss 36 zum Anschluss 26 geführt wird.

Wie aus Figur 1 ersichtlich ist, ist die gesamte Anordnung 10 in sich geschlossen und steril vorgefertigt, d. h. sie ist steril. Es befinden sich im Beutel 12 eine Antikogulanz-Lösung, im Plasmafilter 28 eine Primer-Lösung in Form einer Kochsalzlösung und im dritten Blutbeutel 66 eine Additiv-Lösung. Sämtliche Klemmen 44, 46, 48, 52, 58 und 64 sind geschlossen.

Die Anordnung 10 gemäß Figur 1 wird folgendermaßen betrieben:

Vollblut wird über eine nicht gezeigte Entnahmeleitung dem Vollblutbeutel 12 zugeführt, der anschließend verschlossen bzw. geschweißt wird. Dieses Vollblut wird mindestens 30 Minuten zwischengelagert, um die Abtrennung der Leukozyten zu verbessern.

Im Anschluss daran werden zunächst die Klemmen 44 und 46 geöffnet, so dass das Vollblut durch den Filter 20 strömen kann. Dieses Vollblut gelangt bei geöffneter Klemme 58 in die Blutkammer 32 und verdrängt zunächst die Kochsalzlösung durch die Poren der Membran 30 in die Plasmakammer 34. Der hydrostatische Druck, der sich durch den Abstand a vor dem Vollblutbeutel 12 zum Einlass 26 des Plasmafilter 28 ergibt, drückt Plasma bei geöffneter Klemme 58 durch die Poren der Membran 30 und somit die Primer-Lösung aus dem Plasmaanschluss 38 durch die Leitung 50 zum Abzweig 55, die Leitung 56 und daran in den Auffangbeutel 60, der so dimensioniert ist, dass er die gesamte Primer-Lösung auffangen kann. Sobald gelblich gefärbtes Plasma am Abzweig 55 erscheint, wird die Klemme 58 geschlossen und die Plasmaklemme 52 geöffnet, so dass Plasma in den Plasmabeutel 54 laufen kann. Zugleich wird die Klemme 48 der dritten Leitung 40 geöffnet, so dass Erythrozyten-Konzentrat in den ersten Erythrozyten-Konzentratbeutel fließen kann.

Die Filtration dauert so lange, bis der Vollblutbeutel 12 leer ist. Dabei befindet sich dann erstes Erythrozyten-Konzentrat im zweiten Blutbeutel 42, der mit dem Abstand b unterhalb des Plasmafilters 28 angeordnet ist, während thrombozytenhaltiges Plasma im Plasmabeutel 54 vorliegt, der mit dem Abstand c unterhalb des Plasmafilters 28 angeordnet ist.

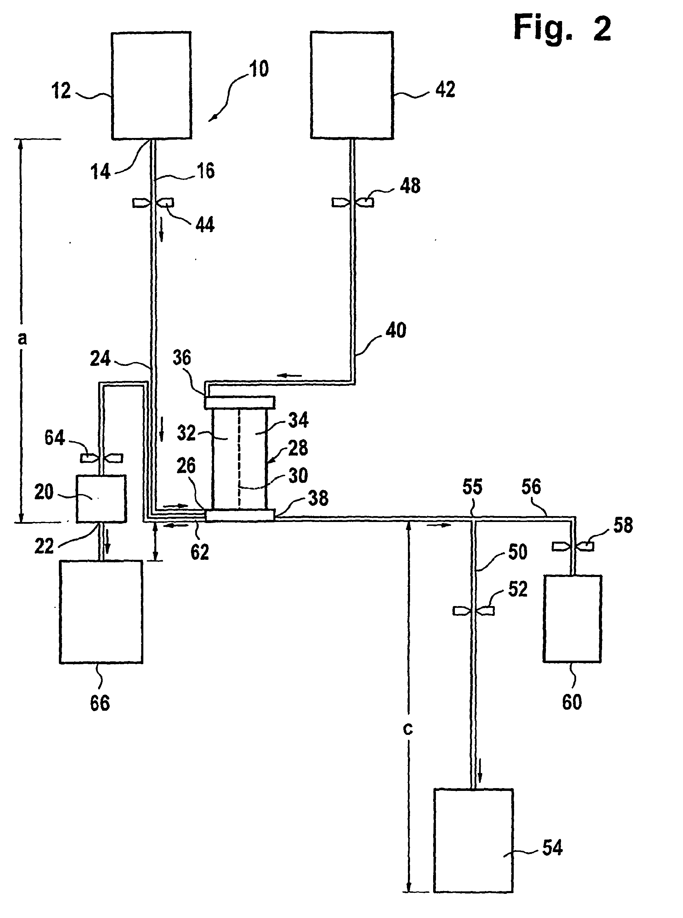

In Figur 2 in die Anordnung 10 in der zweiten Separationsstufe dargestellt, wobei der zweite Blutbeutel 42, der das erste Erythrozyten-Konzentrat enthält, gedreht und in eine Position oberhalb des Plasmafilters 28 angeordnet ist, üblicherweise mit dem gleichen Abstand a wie der Vollblutbeutel 12 zum Plasmafilter 28.

Zusätzlich ist in Figur 2 eine weitere Ausführungsform der Anordnung des Leukozytenfilters 20 dargestellt, der nunmehr nicht in der ersten Leitung 16, sondern vielmehr in der vierten Leitung 62 stromab der Klemme 64 angeordnet ist.

Dabei sind beide Anordnungen des Leukozytenfilters gleichwertig.

In der zweiten Separationsstufe wird zunächst die Zuführungsleitung 16 mit Hilfe der Klemme 46 geschlossen. Zugleich wird die fünfte Leitung 62 mit Hilfe der Klemme 64 geöffnet.

Wie aus Figur 2 ersichtlich ist, befindet sich der zweite Erythrozyten-Beutel 66 etwa mit dem gleichen Abstand b wie der erste Erythrozyten-Beutel 42 in der ersten Separationsstufe unterhalb des Plasmafilters 28.

Es erfolgt nunmehr erneut eine Plasma-Separation in Gegenrichtung von dem Anschluss 36 zum Anschluss 26 und von dort durch die Leitung 62 in den Beutel 66.

Sobald das System leer gelaufen ist, werden der Plasma-Beutel 54 und der das zweite Erythrozyten-Konzentrat enthaltene Beutel 66 abgeschweißt und der weiteren Verwendung zugeführt. Der Rest wird verworfen.

In einer weiteren Ausführungsform lassen sich die manuell betätigbaren Klemmen auch durch elektrisch betätigte Klemmen ersetzen, die vorbestimmt aktiviert so werden, wie dies vorstehend erläutert ist. Dabei lässt sich über einen Strömungssensor das Ende der Strömung durch die Blutbeutel 12 bzw. 42 im zweiten Separationsschritt detektieren. Hierdurch lassen sich die darauf folgenden Aktionen, wie vorstehend erläutert, auslösen. Am Ende des ersten Separationsvorgangs kann der Beutel 42 von der unteren Position in die obere Position befördert werden, wobei zugleich die Klemmen in der oben beschriebenen Weise betätigt werden. Infolgedessen lässt sich die gesamte Anordnung 10 auch vollautomatisch betreiben.

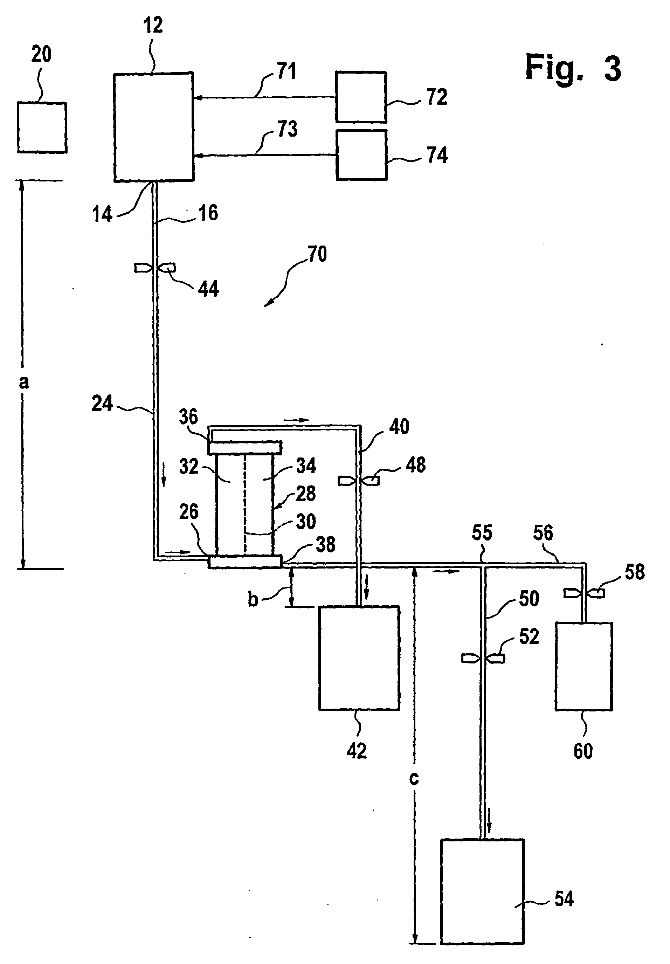

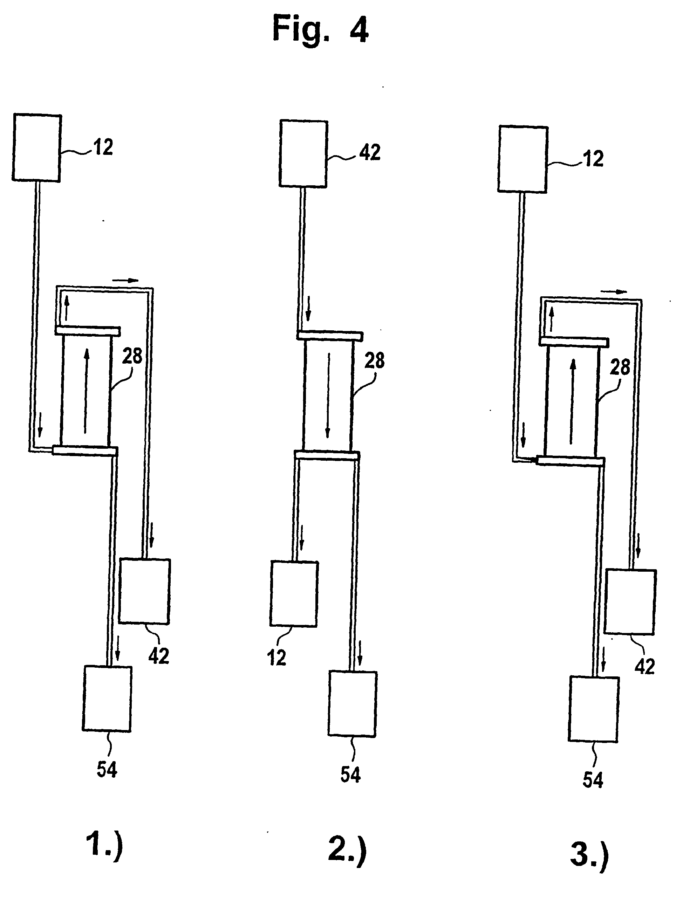

In Figur 3 ist eine weitere Anordnung 70 zur Trennung von Vollblut im Plasma und Erythozytenkonzentrat als weitere unabhängige Ausführungsform gezeigt, die weitgehend der Anordnung 10 entspricht, so dass die Bezugszeichen der einzelnen Komponenten beibehalten worden sind. Die Anordnung 70 unterscheidet sich jedoch im wesentlichen dadurch, dass das Filter 20 nicht mehr in die Leitung 16 eingeschaltet ist, sondern sich vielmehr stromauf des Vollblutbeutels 12 befindet, was in Figur 3 symbolisch dadurch dargestellt ist, dass der Filter 20 nicht mit dem Vollblutbeutel 12 verbunden ist. Es handelt sich hier also um eine außerhalb des Systems erfolgende Filtration zur Entfernung von Leukozyten. Infolgedessen erübrigt sich auch die Klemme 46 und die Leitungsanordnung 62-66, denn die beiden Beutel 12 und 42 können wechselseitig auf- oder abgehängt werden. Dieser Sachverhalt ist in Figur 4 dargestellt, die drei Filtrationsschritte unter Einsatz von zwei Beuteln gemäss Figur 3 zeigt.

Figur 4 erläutert die einzelnen Filtrationsschritte unter Herauf- oder Herabsetzen der Beutel 12 und 54, wobei die Flussrichtung im Plasmafilter 28 durch einen Pfeil dargestellt ist.

Des weiteren kann die Anordnung 70 gemäss Figur 3 mit einem Beutel 72 versehen sein, der eine Additivlösung enthält. Dieser Beutel 72 ist über eine abschließbare Leitung 71 mit dem Vollblutbeutel 12 verbunden. Am Ende des Filtrationsvorgangs kann also das im Vollblutbeutel 12 enthaltene Erythrozytenkonzentrat mit der Additivlösung auf einen vorbestimmten Hämatokritwert eingestellt werden.

Schließlich ist in einer weiteren Ausführungsform eine Kochsalzlösung in einem weiteren Beutel 74 vorgesehen, der über eine Leitung 73 mit dem Vollblutbeutel verbunden ist. Diese Lösung kann zur Wäsche des Erythrozytenkonzentrats im Vollblutbeutel 12 eingesetzt werden, wodurch der Plasmagehalt gesenkt werden kann. Wie bereits vorstehend beschrieben, wird Kochsalzlösung in den Vollblutbeutel 12 über die Leitung 73 zugeführt und anschließend mit dem Eryhtrozytenkonzentrat vermischt. Im Anschluss daran wird eine erneute Filtration per Schwerkraft durch das Plasmafilter 28 durchgeführt. Danach wird Additivlösung dem Konzentrat - wie vorstehend beschrieben - zugesetzt.

### Beispiel

552 g Vollblut (Hämatokrit 40,8 %), das 30 Minuten nach Abnahme von einem Spender gelagert worden ist, werden bei 26 ° C der Plasma-Separation unterzogen.

Dabei wird das Vollblut zunächst durch einen Leukozytenfilter außerhalb des Filtrationssystems (vgl. Figur 3) von Leukozyten befreit. Im Anschluss daran wird dann die Plasma-Separation bei Temperaturen von 32-26 °C durchgeführt.

Die Laufzeit beträgt etwa 10 ± 5 Minuten, bis die erste Separation beendet ist. Man erhält einen Hämatokrit von etwa 55-65 % nach der ersten Trennstufe.

Der Abstand a beträgt bis zu 100 cm (= 1 m WS.). Klein b ist = 10-20 cm und c = 85-100 cm.

Danach werden die Beutel gedreht und die Filtrationsrichtung umgekehrt. Es beginnt also die zweite Phase. Diese ist nach 15 ± 5 Minuten abgeschlossen. Es wird ein Hämatokrit von etwa 65-75 % erhalten.

Mit einem weiteren Durchgang kann der Hämatokrit auf 80-85 erhöht werden. Man erhält ein Netto-Gewicht des Erythrozytenkonzentrats von ca. 180-200 g.

## Patentansprüche

1. Verfahren zum Separieren von Vollblut unter Schwerkraft, bei dem man
a) Vollblut in ein erstes Erythrozyten-Konzentrat und in eine erste, thrombozytenhaltige Plasmafraktion durch Filtration auftrennt, wobei der Hämatokrit des erhaltenen Erythrozytenkonzentrats mindestens 50% beträgt,
b) das erste Erythrozyten-Konzentrat erneut einer Plasmafiltration unter Erhöhung des Hämatokrits auf mindestens 70 % unterwirft und die zweite, thrombozytenhaltige Plasmafraktion der ersten Plasmafraktion zuführt, ggf. eine dritte Plasmafiltration durchführt,
c) das nach der Filtration erhaltene Erythrozyten-Konzentrat mit einer Additivlösung vermischt und
d) das Vollblut oder die Erythroztenkonzentrate durch Filtration von Mikroaggregaten und Leukozyten befreit.

2. Verfahren nach Anspruch 1, **dadurch gekennzeichnet, dass** man die Plasmafiltration mit einem hydrostatischen Druck von 1,5 - 2,5 m, vorzugsweise 1 m Wassersäule durchführt.

3. Verfahren nach Anspruch 1 oder 2, **dadurch gekennzeichnet, dass** man in Stufe a) das Vollblut aus einem ersten Beutel in die Blutkammer eines Plasmafilters fördert, dort unter Filtration Plasma, das in einem Plasmabeutel aufgefangen wird, ein erstes Erythrozyten-Konzentrat, das in einem zweiten Blutbeutel aufgefangen wird, gewinnt und in der Stufe b) den zweiten Blutbeutel mit einem dritten Blutbeutel ebenfalls durch die Blutkammer hindurch, jedoch in umgekehrter Strömungsrichtung verbindet und eine zweite Plasmafiltration durchführt, wobei der erste Blutbeutel abgeklemmt wird, das abgetrennte Plasma im Plasmabeutel und die zweite Erythrozytenfraktion im dritten Blutbeutel auffängt.

4. Verfahren nach einem der Ansprüche 1 bis 3, **dadurch gekennzeichnet, dass** man im Plasmafilter vorgelegte Fülllösungen zu Beginn der Plasmafiltration abtrennt, vorzugsweise in einem weiteren Auffangbeutel, der mit dem Auslass der Plasmakammer in Strömungsverbindung steht.

5. Verfahren nach einem der Ansprüche 1 bis 3, **dadurch gekennzeichnet, dass** man das zweite Erythrozyten-Konzentrat mit PAGGS-Mannitol-Lösung als Additivlösung verdünnt.

6. Verfahren nach einem der Ansprüche 1 bis 5, **dadurch gekennzeichnet, dass** man die thrombozytenhaltige Plasmafraktion durch Filtration in zellfreies Plasma und ein thrombozytenhaltiges Plasmakonzentrat auftrennt.

7. Vorrichtung zum Separieren von Vollblut in ein Erythrozyten-Konzentrat und Plasmalösung unter Schwerkraft, aufweisend
einen Vollblut aufnehmenden ersten Blutbeutel (12),
einen Plasmafilter (28), der durch eine Erythrozyten zurückhaltende und Thrombozyten durchlässige Membran (30) in eine Blutkammer (32) und in eine Plasmakammer (34) geteilt ist, wobei die Blutkammer (32) einen ersten Anschluss (26) und einen zweiten Anschluss (36) und die Plasmakammer (34) einen weiteren Anschluss (38) aufweist,
eine Schlauchleitung (16, 24), die vom ersten Beutel (12) abgeht und mit dem ersten Anschluss (26) der Blutkammer (32) verbunden ist,
einen Mikroaggregate und Leukozyten entfernenden Leukozytenfilter (20), eine dritte Schlauchleitung (40), die vom zweiten Anschluss (36) der Blutkammer (32) abgeht und mit einem zweiten Blutbeutel (42) verbunden ist,
eine vierte Schlauchleitung, die vom Auslass (38) der Plasmakammer (34) abgeht und mit einem Plasmabeutel (54) verbunden ist,
sowie
eine Absperreinrichtung (44) für die Leitung (16) und eine zweite Absperreinrichtung (48) für die dritte Leitung (40).

8. Vorrichtung nach Anspruch 7, **dadurch gekennzeichnet, dass** der Leukozytenfilter (20) in die Schlauchleitung (16) eingeschaltet ist, stromab des Leukozytenfilters (20) eine Absperreinrichtung (46) in die Leitung (24) eingeschaltet ist und eine fünfte Schlauchleitung (62) vom ersten Anschluss (26) der Blutkammer (32) abgeht und mit einem dritten Blutbeutel (66) verbunden ist.

9. Vorrichtung nach Anspruch 7 oder 8, **dadurch gekennzeichnet, dass** der Auslass (38) des Plasmafilters (28) mit einem Auffangbeutel (60) für eine Füllflüssigkeit des Plasmafilters (28) über eine Abzweigleitung (56) in Strömungsverbindung steht und eine zweite Klemme (58) zum Absperren der Abzweigleitung (56) sowie eine dritte Klemme (52) zum Absperren der vierten Leitung (50) vorgesehen sind.

10. Vorrichtung nach Anspruch 7-9, **dadurch gekennzeichnet, dass** der dritte Blutbeutel (66) eine Additivlösung zum Vermischen des zweiten Erythrozytenkonzentrats aufweist.

11. Vorrichtung nach einem der Ansprüche 7-10, **dadurch gekennzeichnet, dass** der Plasmafilter (28) eine mittlere Porengröße von 1-2 µm zum Abfiltern von Plasma, Proteinen und Thrombozyten aufweist.

12. Vorrichtung nach einem der Ansprüche 7-11, des weiteren aufweisend einen Thrombozytenfilter. mit einer mittleren Porengröße von 0,03-0,4 µm zum Abfiltrieren eines Thrombozytenkonzentrats aus der thrombozytenhaltigen Plasmafraktion.

## Claims

1. A method for separating full blood under gravitational force, wherein
a) full blood is separated by filtration into a first erythrocyte concentrate and into a first, thrombocyte-containing plasma fraction, the haematocrit of the obtained erythrocyte concentrate amounting to at least 50%,
b) the first erythrocyte concentrate is again subjected to a plasma filtration with an increase in the haematocrit to at least 70% and the second, thrombocyte-containing plasma fraction is fed to the first plasma fraction, in that case a third plasma filtration being carried out,
c) the erythrocyte concentrate obtained after the filtration is mixed with an additive solution and
d) the full blood or the erythrocyte concentrates are freed by filtration from microaggregates and leukocytes.

2. The method according to claim 1, **characterised in that** the plasma filtration is carried out at a hydrostatic pressure of 1.5 - 2.5 m, preferably 1 m water column.

3. The method according to claim 1 or 2, **characterised in that** in stage a) the full blood is conveyed from a first bag into the blood chamber of a plasma filter, a first erythrocyte concentrate, which is collected in a second blood bag, is obtained there by filtration of plasma, which is collected in a plasma bag, and in stage b) the second blood bag is connected to a third blood bag likewise through the blood chamber, but in the reverse flow direction, and a second plasma filtration is carried out, whereby the first blood bag is clamped off, the separated plasma being collected in the plasma bag and the second erythrocyte fraction in the third blood bag.

4. The method according to any one of claims 1 to 3, **characterised in that** filling solutions previously placed in the plasma filter are separated at the start of the plasma filtration, preferably in a further collection bag, which is in a fluidic connection with the outlet of the plasma chamber.

5. The method according to any one of claims 1 to 3, **characterised in that** the second erythrocyte concentrate is diluted with PAGGS-Mannitol solution as an additive solution.

6. The method according to any one of claims 1 to 5, **characterised in that** the thrombocyte-containing plasma fraction is separated by filtration into cell-free plasma and a thrombocyte-containing plasma concentrate.

7. A device for separating full blood into an erythrocyte concentrate and plasma solution under gravitational force, comprising
a first blood bag (12) receiving full blood,
a plasma filter (28), which is divided into a blood chamber (32) and into a plasma chamber (34) by a membrane (30) that holds back erythrocytes and lets thrombocytes through, the blood chamber (32) having a first connector (26) and a second connector (36) and the plasma chamber (34) having a further connector (38),
a tube line (16, 24) which extends from the first bag (12) and is connected to the first connector (26) of the blood chamber (32),
a leukocyte filter (20) which removes microaggregates and leukocytes,
a third tube line (40) which extends from the second connector (36) of the blood chamber (32) and is connected to a second blood bag (42),
a fourth tube line which extends from the outlet (38) of the plasma chamber (34) and is connected to a plasma bag (54),
and
a shut-off device (44) for the line (16) and a second shut-off device (48) for the third line (40).

8. The device according to claim 7, **characterised in that** the leukocyte filter (20) is coupled into the tube line (16), a shut-off device (46) is coupled in the line (24) downstream of the leukocyte filter (20) and a fifth tube line (62) extends from the first connector (26) of the blood chamber (32) and is connected to a third blood bag (66).

9. The device according to claim 7 or 8, **characterised in that** the outlet (38) of the plasma filter (28) is in a fluidic connection via a branch line (56) with a collection bag (60) for a filling liquid of the plasma filter (28) and a second clamp (58) for shutting off the branch line (56) and a third clamp (52) for shutting off the fourth line (50) are provided.

10. The device according to claim 7-9, **characterised in that** the third blood bag (66) has an additive solution for mixing with the second erythrocyte concentrate.

11. The device according to any one of claims 7-10, **characterised in that** the plasma filter (28) has a mean pore size of 1-2 µm for filtering of plasma, proteins and thrombocytes.

12. The device according to any one of claims 7-11, further comprising a thrombocyte filter with a mean pore size of 0.03-0.4 µm for filtering of a thrombocyte concentrate from the thrombocyte-containing plasma fraction.

## Revendications

1. Procédé de séparation de sang total par gravité, dans lequel
a) on isole par filtration le sang total présent dans un premier concentré d'érythrocytes et dans une première fraction plasmatique contenant des thrombocytes, l'hématocrite du concentré d'érythrocytes obtenu étant égal à au moins 50 %,
b) le premier concentré d'érythrocytes est soumis à nouveau à une filtration plasmatique avec augmentation de l'hématocrite d'au moins 70 % environ et la deuxième fraction plasmatique contenant des thrombocytes est ajoutée à la première fraction plasmatique, et on procède le cas échéant à une troisième filtration plasmatique,
c) le concentré d'érythrocytes obtenu après la filtration est mélangé à une solution d'additif et
d) le sang total ou le concentré d'érythrocytes est débarrassé par filtration des micro-agrégats et des leucocytes.

2. Procédé selon la revendication 1, **caractérisé en ce qu'**on réalise la filtration plasmatique avec une pression hydrostatique de 1,5 à 2,5 m, de préférence 1 m de colonne d'eau.

3. Procédé selon la revendication 1 ou 2, **caractérisé en ce qu'**on achemine le sang total depuis une première poche dans le compartiment sanguin d'un filtre plasmatique à l'étape a), on obtient par filtration un plasma, qui est recueilli dans une poche de plasma, un premier concentré d'érythrocytes, qui est recueilli dans une deuxième poche de sang, et **en ce qu'**on relie à l'étape b) la deuxième poche de sang avec une troisième poche de sang par l'intermédiaire du compartiment sanguin, mais en sens inverse et **en ce qu'**on réalise une deuxième filtration plasmatique, la première poche de sang étant fermée par une pince, le plasma isolé étant recueilli dans la poche de plasma et la deuxième fraction d'érythrocytes dans la troisième poche de sang.

4. Procédé selon l'une des revendications 1 à 3, **caractérisé en ce qu'**on isole les solutions de remplissage présentées dans le filtre plasmatique au début de la filtration du plasma, de préférence dans une autre poche de stockage qui est en connexion en flux avec la sortie du compartiment plasmatique.

5. Procédé selon l'une des revendications 1 à 3, **caractérisé en ce qu'**on dilue le deuxième concentré d'érythrocytes avec une solution de PAGGS-mannitol comme solution d'additif.

6. Procédé selon l'une des revendications 1 à 5, **caractérisé en ce qu'**on sépare par filtration la fraction plasmatique contenant des thrombocytes en du plasma acellulaire et un concentré plasmatique contenant les thrombocytes.

7. Dispositif de séparation du sang total en un concentré d'érythrocytes et une solution plasmatique par gravité, présentant
une première poche sanguine (12) contenant le sang total,
un filtre plasmatique (28), qui est divisé par une membrane perméable pour les thrombocytes et imperméable pour les érythrocytes (30) en un compartiment sanguin (32) et un compartiment plasmatique (34), le compartiment sanguin (32) présentant une première connexion (26) et une deuxième connexion (36) et le compartiment plasmatique (34) présentant une autre connexion (38),
une conduite flexible (16, 24), qui part de la première poche (12) et qui est reliée à la première connexion (26) du compartiment sanguin (32),
un filtre leucocytaire (20) éliminant les micro-agrégats et les leucocytes,
une troisième conduite flexible (40), qui part de la deuxième connexion (36) du compartiment sanguin (32) et qui est reliée au deuxième compartiment sanguin (42),
une quatrième conduite flexible, qui part de la sortie (38) du compartiment plasmatique (34) et qui est relié à la poche plasmatique (54),
ainsi que
un dispositif de blocage (44) pour la conduite (16) et un deuxième dispositif de blocage (48) pour la troisième conduite (40).

8. Dispositif selon la revendication 7, **caractérisé en ce que** le filtre leucocytaire (20) est activé dans la conduite flexible (16), un dispositif de blocage (46) est activé en aval du filtre leucocytaire (20) dans la conduite (24) et une cinquième conduite flexible (62) part de la première connexion (26) du compartiment sanguin (32) et est reliée à une troisième poche sanguine (66).

9. Dispositif selon la revendication 7 ou 8, **caractérisé en ce que** la sortie (38) du filtre plasmatique (28) est en liaison de flux avec une poche de stockage (60) pour un liquide de remplissage du filtre plasmatique (28) par l'intermédiaire d'une dérivation (56) et **en ce qu'**une deuxième pince (58) est prévue pour bloquer la dérivation (56) ainsi qu'une troisième pince (52) pour bloquer la quatrième conduite (50).

10. Dispositif selon les revendications 7 à 9, **caractérisé en ce que** la troisième poche sanguine (66) présente une solution d'additif en vue d'un mélange avec le deuxième concentré d'érythrocytes.

11. Dispositif selon l'une des revendications 7 à 10, **caractérisé en ce que** le filtre plasmatique (28) présente des pores d'une taille moyenne de 1-2 µm pour filtrer le plasma, les protéines et les thrombocytes.

12. Dispositif selon l'une des revendications 7 à 11 présentant en outre un filtre de thrombocytes avec des pores d'une taille moyenne de 0,03-0,4 µm pour filtrer un concentré thrombocytaire à partir de la fraction plasmatique contenant les thrombocytes.
